# FASCICULE DE BREVET EUROPEEN

(11) **EP 1 404 901 B1**
(45) Date de publication et mention de la délivrance du brevet: **07.09.2005**
(21) Numéro de dépôt: 02755081.3
(22) Date de dépôt: 21.06.2002
(51) Int. Cl.: C30B 7/00

(54) **PROCEDE DE PREPARATION DE NANOTUBULES ORGANIQUES, NOTAMMENT D'ACIDE LITHOCHOLIQUE, ET LEURS UTILISATIONS**
VERFAHREN ZUR HERSTELLUNG ORGANISCHEN NANORÖHRCHEN, INSBESONDERE AUS LITHOCHOLSÄURE UND IHRE ANWENDUNGEN
METHOD FOR PREPARING ORGANIC, IN PARTICULAR LITHOCHOLIC ACID, NANOTUBES AND USES THEREOF

(30) Priorité: 12.07.2001 FR 0109299
(43) Date de publication de la demande: 07.04.2004
(73) Titulaire: COMMISSARIAT A L'ENERGIE ATOMIQUE, 75752 Paris Cédex 15 (FR); CENTRE NATIONAL DE LA RECHERCHE SCIENTIFIQUE, 75794 Paris Cedex 16 (FR)
(72) Inventeur: TERECH, Pierre, F-38500 Saint-Cassien (FR)
(74) Mandataire: Poulin, Gérard
(86) Numéro de dépôt international: PCT/FR2002/002166
(87) Numéro de publication internationale: WO 2003/007323

(56) Documents cités:
- PATENT ABSTRACTS OF JAPAN vol. 1999, no. 14, 22 décembre 1999 (1999-12-22) & JP 11 246551 A (JAPAN SCIENCE &TECHNOLOGY CORP), 14 septembre 1999 (1999-09-14)
- BONG D T ET AL: "ORGANIC NANOTUBES" ANGEWANDTE CHEMIE. INTERNATIONAL EDITION, VERLAG CHEMIE. WEINHEIM, DE, vol. 40, no. 6, 2001, pages 988-1011, XP001044098 ISSN: 0570-0833 cité dans la demande
- SCHNUR J M ET AL: "SELF-ASSEMBLING PHOSPHOLIPID TUBULES" ADVANCED MATERIALS, VCH VERLAGSGESELLSCHAFT, WEINHEIM, DE, vol. 6, no. 12, 1 décembre 1994 (1994-12-01), pages 971-974, XP000480054 ISSN: 0935-9648 cité dans la demande
- SCHNUR J M: "LIPID TUBULES: A PARADIGM FOR MOLECULARLY ENGINEERED STRUCTURES" SCIENCE, AMERICAN ASSOCIATION FOR THE ADVANCEMENT OF SCIENCE,, US, vol. 262, no. 5140, 10 décembre 1993 (1993-12-10), pages 1669-1676, XP002056380 ISSN: 0036-8075 cité dans la demande

## Description

### Domaine technique

La présente invention a pour objet la fabrication de nanotubules organiques.

De tels nanotubules peuvent trouver diverses applications, notamment dans le secteur des biocapteurs et des transporteurs de principes actifs, et dans le secteur de la nano-électronique.

### Etat de la technique antérieure

L'auto-assemblage spontané de petites molécules en nanostructures constitue une méthode alternative douce et simple pour fabriquer des matériaux dont le siège de l'activité se situe également à l'échelle nanoscopique. Deux grands secteurs d'application peuvent être concernés par ces structures nanotubulaires, comme il est décrit par Bong, D.T., Clark, T.D. Granja, J.R. and Ghadiri, M.R. (2001), Angew. Chem. Int. Ed., 40, 988-1011, [1].

Le premier secteur est celui de la chimie où ces structures nanotubulaires peuvent être utilisées dans des biocapteurs ou pour le transport de produits bioactifs.

Le second secteur est celui de l'électronique et de l'électro-optique, comme il est décrit par Trau M., Yao N., Kim E., Xia Y., Whitesides, G.M. and Aksay I.A. (1997), Nature, 390, 674-676 [2].

De façon schématique, on peut résumer le fonctionnement d'applications dans le premier secteur en indiquant que les tubules peuvent être utilisées telles quelles si le milieu où elles existent est compatible avec celui à sonder ou à traiter. La diffusion limitée par la taille du coeur creux de la tubule peut constituer le principe de fonctionnement d'un des types d'applications. Si ce coeur peut être fonctionnalisé de façon adéquate pour présenter des propriétés de conduction électronique et/ou ionique comme il est décrit par Van Nostrum, C.F. (1996), Adv. Mater., 8, 1027-1030 [3], d'autres applications sont possibles.

Le deuxième champ d'application passe par la rigidification des tubules pour en faire une réplique solide qui à son tour peut être utilisée pour les applications mentionnées ci-dessus, mais aussi pour la fabrication de matériaux composites électro-actifs. Cette étape de rigidification peut être réalisée par métallisation des structures ou minéralisation à la silice. La métallisation permet en même temps d'introduire les propriétés électromagnétiques intéressant les applications. La minéralisation est une méthode de chimie douce permettant de transiter du domaine organique vers celui du minéral, on peut retrouver les champs d'application des nanotubes de carbone tels que décrits par Dekker, C. (1999), Physics Today, 52, 22-28 [4] avec les tubules rigidifiées décrites par Schnur, J.M. (1993), Science, 262, 1669-1676 [5], et Schnur, J.M. and Shashidhar, R. (1994), Adv. Mater., 6, 971-974 [6].

En ce qui concerne les applications hors du domaine électronique et opto-électronique, on peut aussi citer la catalyse, la séparation biomoléculaire, et la préparation de membranes poreuses à trous calibrés pour des applications de filtration où l'on fait une réplique par polymérisation autour des structures fibrillaires, comme il est décrit par Gu, W., Lu, L. Chapman, G.B. and Weiss, R.G. (1997) J. Chem. Soc., Chem. Commun., 543-544 [7]. D'autres structures à base de silice ont été décrites par Jung, H. J., Amaike, M. and Shinkai S. (2000), J. Chem. Soc., Chem. Commun., 2343-2344 [8].

Bien souvent, l'efficacité d'un système formé à partir d'éléments actifs individuels nanoscopiques sera amplifié si ces éléments sont orientés et alignés.

Actuellement, les systèmes chimiques conduisant à la formation de tubules appartiennent aux familles chimiques suivantes décrites dans la référence [1] :
- peptides linéaires ou cycliques,
- macrocycles,
- cyclodextrines,
- lipides, et
- copolymères blocs.

Avec ces molécules, le diamètre intérieur des tubules n'est pas toujours dans le domaine nanométrique, compris entre quelques dizaines d'Angströms, (1/10 nanomètre, soit 10⁻⁹ m) et quelques centaines d'Angströms. Par ailleurs, ces molécules résultent de synthèses chimiques parfois complexes.

Les procédés de fabrication font souvent intervenir une étape de chauffage et parfois plusieurs constituants.

Par ailleurs, l'orientation des tubules suppose qu'elles interagissent faiblement entre elles et l'utilisation de champs électriques est alors nécessaire. Dans d'autres cas, on obtient l'orientation en utilisant des microcapillaires, comme il est décrit dans la référence [7].

Ainsi, dans la référence [2], des tubules peuvent être obtenues à partir de l'auto-assemblage de fibres pleines de chlorure de cétyltryméthylammonium. Leur orientation est obtenue en appliquant un champ électrique dans des microcapillaires où la réaction chimique de formation des tubules et une minéralisation simultanée sont conduites. La procédure d'alignement est donc ici complexe. Le diamètre intérieur de la tubule résultante est fixé par le diamètre des fibres pleines initiales, le diamètre extérieur ne peut être contrôlé avec précision. Le produit de départ est un tensio-actif de synthèse.

Dans la référence [8], on obtient des tubules de silice par une technique de polymérisation sol-gel sur des structures organiques.

Dans les références [5] et [6], des tubules peuvent être formées à partir du phospholipide polymérisable 1,2-bis(tricosa)-10, 12-10 diynoyl)sn-glycéro-3-phosphocholine qui n'est pas une structure chimique simple. Le diamètre interne est de l'ordre de 0,2 à 0,7 µm et ne situe pas dans le domaine nanoscopique.

Les procédés et les molécules utilisés jusqu'à présent pour la formation de tubules présentent donc certains inconvénients.

En effet, ils ne répondent pas à l'ensemble des caractéristiques recherchées pour l'obtention de telles structures, qui sont :
1) la facilité d'obtention du produit de départ afin d'éviter des synthèses chimiques complexes et coûteuses ;
2) la facilité d'obtention des tubules à partir du produit de départ ;
3) l'homogénéité des nanostructures formées, c'est-à-dire des dimensions du diamètre extérieur et du trou interne qui doivent être parfaitement calibrées ;
4) la stabilité des nanostructures formées ; et
5) la facilité d'obtention de tubules alignées parallèlement les uns aux autres.

### Exposé de l'invention

La présente invention a précisément pour objet un procédé d'obtention de tubules dont les dimensions du diamètre extérieur et du diamètre intérieur, soit du trou interne, sont parfaitement calibrées dans le domaine nanométrique, utilisant comme produit de départ un produit naturel secrété par la bile et qui ne nécessite aucune opération de synthèse organique supplémentaire, et permettant d'obtenir facilement des nanotubules en milieu aqueux.

Selon l'invention, le procédé de préparation de nanotubules organiques en milieu aqueux comprend les étapes suivantes :
a) préparer une solution aqueuse basique ayant un pH de 10 à 14, de préférence de 12 à 13,5.
b) ajouter à la solution un composé organique de formule : 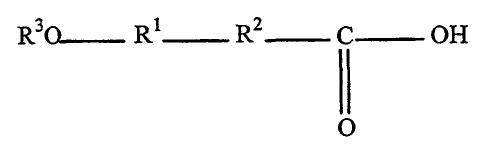 dans laquelle R¹ est un radical polycyclique à noyaux condensés en C₁₇ à C₂₀ comportant éventuellement un ou plusieurs substituants alkyle en C₁ à C₂₀, R² est un groupe alkylène linéaire ou ramifié en C₃ à C₂₀, et R³ représente un atome d'hydrogène, un groupe alkyle en C₁ à C₂₀ ou un groupe aromatique en C₆ à C₃₀, et
c) soumettre la solution à une agitation pendant une durée suffisante pour former des tubules stables du composé organique dans la solution.

Le composé organique de départ utilisé dans l'invention comprend ainsi un groupe carboxylique et un groupe hydroxyle séparés par un radical polycyclique à noyaux condensés éventuellement substitué et un groupe alkylène qui permet de former facilement des tubules de dimensions calibrées.

Le groupe R² de ce composé est un groupe alkylène qui peut comporter de 3 à 20 atomes de carbone, de préférence de 3 à 8 atomes de carbone.

A titre d'exemple d'un tel groupe, on peut citer le groupe :

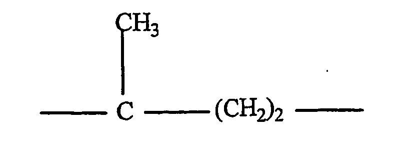

Dans ces composés, le groupe R¹ est un groupe polycyclique à noyaux condensés, qui peut être dérivé de produits naturels tels que les stéroïdes.

Dans ce composé, le choix des groupes R¹ et R² permet en particulier de régler les dimensions des tubules, soit leurs diamètres interne et externe.

A titre d'exemples de tels groupes polycycliques, on peut citer le groupe de formule : 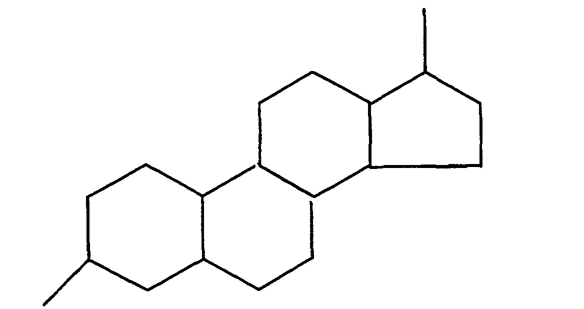 qui peut comporter un ou plusieurs substituants alkyle en C₁ à C₂₀ environ.

Selon l'invention, R³ peut représenter un atome d'hydrogène, un groupe alkyle, linéaire ou ramifié, en C₁ à C₂₀, ou un groupe aromatique en C₆ à C₃₀.

On entend par groupe aromatique un groupe possédant un ou plusieurs noyaux benzéniques, naphtaléniques, anthracéniques, et pouvant comporter éventuellement des hétéroatomes tels que O, S et N.

A titre d'exemples de tels groupes aromatiques, on peut citer les groupes dérivés de l'anthracène, du benzène et du naphtalène comme les groupes anthryle, benzyle et naphtyle, et les groupes dérivés de l'azobenzène.

De préférence, selon l'invention, le composé organique de départ est l'acide lithocholique de formule :

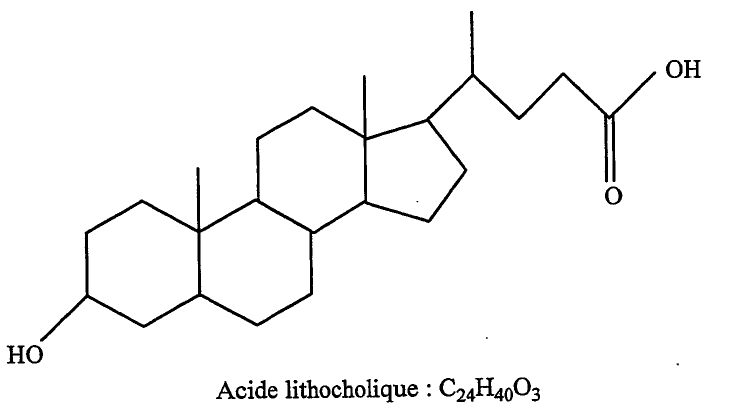

Selon l'invention, cet acide qui est normalement insoluble dans l'eau à pH neutre ou acide, est solubilisé sous forme de nanotubules stables en utilisant un milieu basique ayant un pH ajusté de façon appropriée. Par simple agitation du milieu, on obtient des nanotubules dont les dimensions sont monodisperses et constituent une signature du constituant initial. Les tubules peuvent être orientées par simple cisaillement de la solution.

Ainsi, le procédé de l'invention ne nécessite pas de chauffage, et utilise un produit de départ naturel qui ne nécessite aucune opération de synthèse organique supplémentaire ; il ne nécessite pas davantage l'utilisation de champs électriques ou de microgravures pour orienter les tubules ; il répond donc à l'ensemble des caractéristiques recherchées pour l'obtention de tubules.

Selon l'invention, le milieu aqueux est un milieu basique ayant de préférence un pH de 12 à 13,5. On peut utiliser une solution aqueuse d'hydroxyde de sodium. Lorsque le composé de départ est l'acide lithocholique, on utilise de préférence une solution d'hydroxyde de sodium ayant un pH d'environ 12,5.

Le réglage du pH de ce milieu ainsi que la nature de la base utilisée (force ionique, contre-ions) et la concentration en composés organiques constituent également un moyen de réglage de l'amplitude des interactions entre tubules.

De préférence, cette concentration en composé organique est de 0,01% à 20% en poids.

Selon l'invention, le type de radical polycyclique R₁ et le milieu aqueux (eau légère, eau lourde) jouent également un rôle sur les caractéristiques structurales des tubules.

L'invention a encore pour objet l'utilisation des nanotubules organiques obtenues par ce procédé, pour la fabrication de nanotubes inorganiques par minéralisation, pour la fabrication de cathodes à émission de champ par métallisation des nanotubes ou encore pour la fabrication de biocapteurs.

D'autres caractéristiques et avantages de l'invention apparaîtront mieux à la lecture de la description qui suit, donnés bien entendu à titre illustratif et non limitatif, en référence aux dessins annexés.

### Brève description des dessins

La figure 1 illustre la diffusion typique de rayons X d'un système de tubules d'acide lithocholique en milieu basique.
La figure 2 est une micrographie électronique de tubules d'acide lithocholique.
La figure 3 illustre les courbes de contrainte σ (en Pa) en fonction du taux de cisaillement 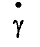 ou dγ/dt (en s⁻¹) de deux solutions de tubules d'acide lithocholique.

### Exposé détaillé d'un mode de réalisation

On décrit ci-après un exemple de préparation de tubules d'acide lithocholique.

On prépare tout d'abord une solution aqueuse basique ayant un pH d'environ 12,5 en ajoutant 0,125 g d'hydroxyde de sodium à 100 g d'eau. On ajoute alors 0,3 g d'acide lithocholique à la solution obtenue. On soumet l'ensemble à une agitation au moyen d'un agitateur magnétique pendant environ une demi-heure et on obtient ainsi une solution de tubules à la concentration de 0,3% en poids.

On caractérise la structure des tubules par l'utilisation complémentaire de la diffusion centrale de rayons X et de neutrons (espace réciproque des moments de transfert de nombres d'onde) et par microscopie électronique en transmission (espace réel des trois dimensions spatiales).

La diffusion centrale de rayons X a l'avantage d'irradier un volume important d'échantillon et donc d'obtenir une réponse qui est une moyenne statistique des nanostructures sondées sur ce volume. La réponse est une courbe de l'intensité diffusée en fonction de l'angle de diffusion qui présente jusqu'à sept oscillations et une décroissance caractéristiques.

La modélisation théorique permet de rendre compte de ces éléments si on utilise un modèle géométrique de tubes creux de diamètre extérieur de 520 Angströms et de diamètre intérieur de 490 Angströms.

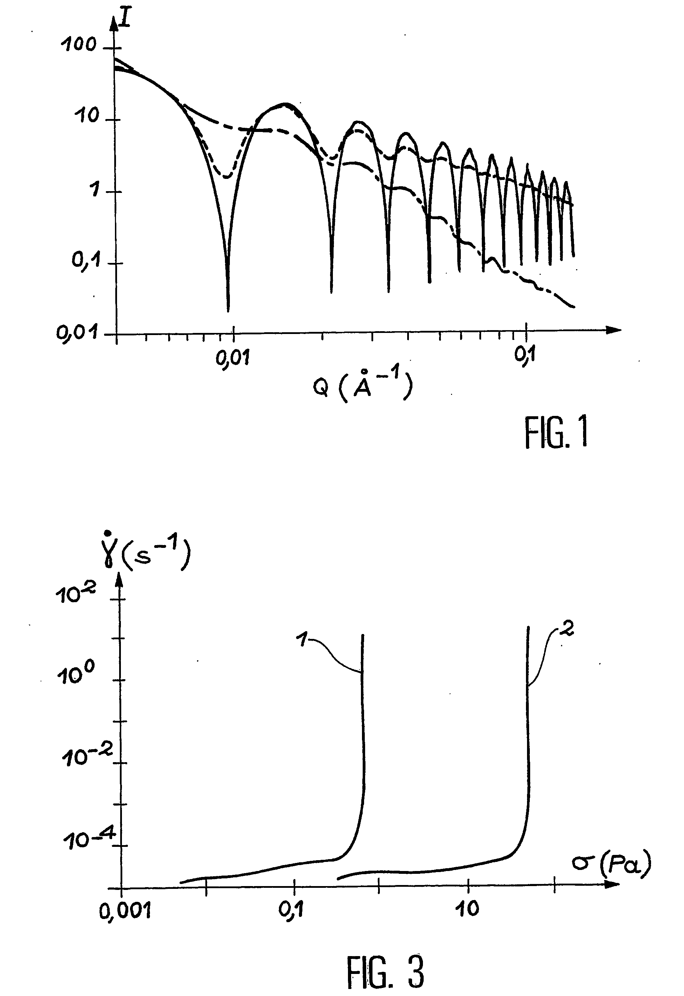

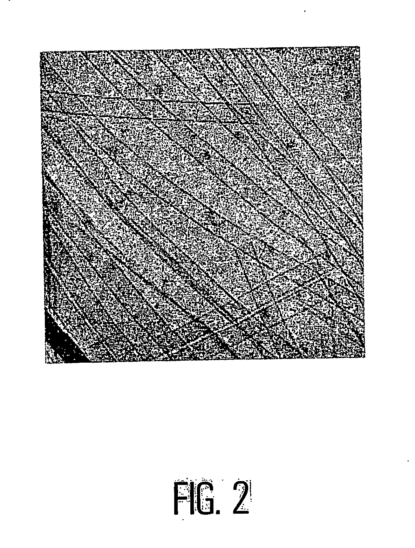

La figure 1 illustre la diffusion typique (intensité diffusée en fonction de l'angle de diffusion) de rayons X du système de tubules.

On distingue sur cette figure plusieurs oscillations expérimentales (courbe en traits mixtes) que le modèle théorique simplifié d'un cylindre creux (courbes en traits pleins et en tirets) reproduit dans leurs positions.

La confirmation sans ambiguïté de l'existence de ces tubules est apportée par des clichés de microscopie électronique en transmission. L'appareillage et le protocole expérimental utilisés sont choisis pour une observation directe des structures sans manipulation du système. On utilise le contraste électronique du système déposé en couche très mince (environ 3000 Angströms) sur une platine réfrigérée d'un microscope spécialisé. Ce procédé est décrit par T. Ruiz, I. Erk et J. Lepault dans Biol. Cell, 1994, 80, p. 203 à 210 [9], pour observer des structures biologiques fragiles.

La figure 2 est une micrographie électronique montrant la présence quasi-exclusive de longs cylindres creux. Sur cette figure, on distingue parfaitement les parois et les extrémités de ces tubules. Les diamètres extérieur et intérieur sont respectivement de 520 Angströms et 490 Angströms.

On examine maintenant les possibilités d'orientation des tubules dans le cas de la solution de tubules à 0,3% et dans le cas d'une solution de tubules à une concentration de 2,4% obtenue de la même façon.

Dans ce but, on soumet la solution aqueuse contenant les tubules à une contrainte de cisaillement ou d'élongation (γ) pour orienter les tubules.

La valeur de cette contrainte minimale de cisaillement est faible, typiquement d'environ 100 s⁻¹ pour une concentration d'environ 2%. Des résultats supérieurs sont obtenus à partir de fibres étirées (élongation) d'une solution très visqueuse plus concentrée (7%). La caractérisation est faite par diffusion centrale de rayons X.

Dans ce but, on place un échantillon de tubules dans l'entrefer de 1 mm formé par deux cylindres coaxiaux dont la rotation de l'élément extérieur génère un cisaillement dans l'entrefer. L'entrefer est frappé par un faisceau de rayons X dont l'intensité diffusée par un détecteur bidimensionnel permet d'analyser le degré d'orientation des tubules. Si les tubules sont distribuées au hasard dans le volume de l'échantillon, le signal de diffusion se manifeste sur le détecteur 2d par des lignes d'iso-intensité qui sont circulaires. Dans cette configuration au repos, on retrouve alors la signature de diffusion avec ses oscillations caractéristiques mentionnées ci-dessus.

Lorsque l'échantillon est étiré, les positions des tubules deviennent corrélées et le signal de diffusion se déforme plus ou moins fortement selon le degré d'orientation. On passe ainsi de lignes d'iso-intensité circulaires à des lignes elliptiques puis à une ligne joignant des tâches de diffusion, c'est ce que l'on observe avec la solution alcaline de tubules.

La capacité d'orientation peut également être appréhendée ou contrôlée par des mesures plus traditionnelles de visco-élasticité.

Dans ce cas, on mesure, quelle que soit la concentration utilisée, le niveau d'interactions des tubules entre elles, soit les courbes de contrainte en fonction du taux de cisaillement, et les modifications de viscosité lorsque le taux de cisaillement augmente. Dans ces conditions, on observe une fluidification du système due à ces effets d'orientation. Les mesures peuvent être réalisées avec un simple rhéomètre.

Les nanotubules sont identifiables par leurs dimensions (diamètres extérieur, intérieur et épaisseur de la paroi) et leurs caractéristiques physico-chimiques, en particulier les paramètres décrivant leur visco-élasticité (courbes d'écoulement, contraintes de seuil, élasticité et viscosité).

La figure 3 représente un exemple d'empreinte rhéologique du système et illustre les courbes de contrainte en fonction du taux de cisaillement, pour deux solutions de tubules ayant une concentration de 0,3% (courbe 1) et une concentration de 2,4% (courbe 2). On obtient ainsi les contraintes de seuil (σ1 et σ2) des deux solutions. Les dimensions transverses des tubules et leurs caractéristiques rhéologiques sont fixées par la formule chimique du constituant l'acide lithocholique et du type de liquide utilisé.

### REFERENCES CITEES

**[1]** Bong, D.T., Clark, T.D. Granja, J.R. and Ghadiri, M.R. (2001), Angew. Chem. Int. Ed., 40, 988-1011,
**[2]** Trau M., Yao N., Kim E., Xia Y., Whitesides, G.M. and Aksay I.A. (1997), Nature, 390, 674-676,
**[3]** Van Nostrum, C.F. (1996), Adv. Mater., 8, 1027-1030,
**[4]** Dekker, C. (1999), Physics Today, 52, 22-28,
**[5]** Schnur, J.M. (1993), Science, 262, 1669-1676,
**[6]** Schnur, J.M. and Shashidhar, R. (1994), Adv. Mater., 6, 971-974,
**[7]** Gu, W., Lu, L. Chapman, G.B. and Weiss, R.G. (1997) J. Chem. Soc., Chem. Commun., 543-544,
**[8]** Jung, H.J., Amaike, M. and Shinkai, S. (2000), J. Chem. Soc., Chem., Commun., 2343-2344,
**[9]** Ruiz et al. dans Biol. Cell, 1994, 80, p. 203 à 210,

## Revendications

1. Procédé de préparation de nanotubules organiques en milieu aqueux, comprenant les étapes suivantes :
a) préparer une solution aqueuse basique ayant un pH de 12 à 14, de préférence de 12 à 13,5,
b) ajouter à la solution un composé organique de formule : 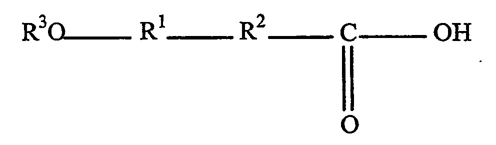 dans laquelle R¹ est un radical polycyclique à noyaux condensés en C₁₇ à C₂₀ comportant éventuellement un ou plusieurs substituants alkyle en C₁ à C₂₀, R² est un groupe alkylène linéaire ou ramifié en C₃ à C₂₀, et R³ représente un atome d'hydrogène, un groupe alkyle en C₁ à C₂₀ ou un groupe aromatique en C₆ à C₃₀, et
c) soumettre la solution à une agitation pendant une durée suffisante pour former des tubules stables du composé organique dans la solution.

2. Procédé selon la revendication 1 dans lequel R² est le groupe :

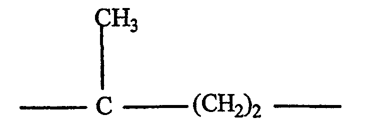

3. Procédé selon l'une quelconque des revendications 1 et 2, dans lequel R¹ est un groupe polycyclique de formule : 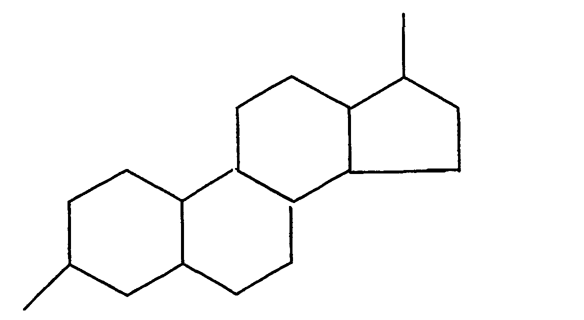 qui peut comporter un ou plusieurs substituants alkyle en C₁ à C₂₀.

4. Procédé selon la revendication 1 dans lequel le composé organique est l'acide lithocholique de formule :

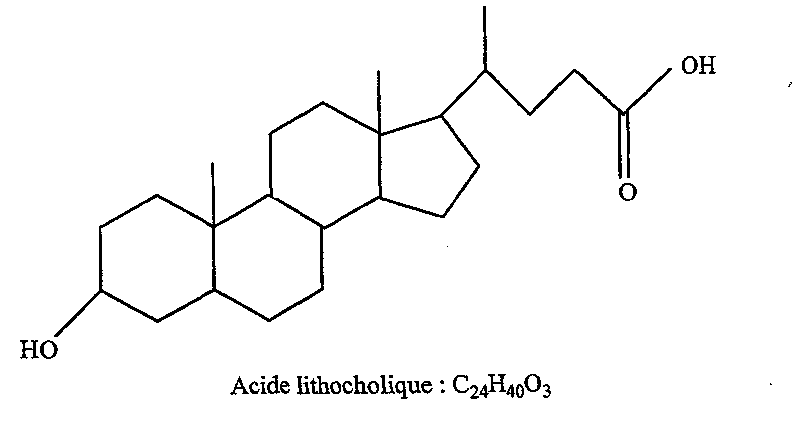

5. Procédé selon l'une quelconque des revendications 1 à 4, dans lequel la solution aqueuse est une solution d'hydroxyde de sodium.

6. Procédé selon la revendication 4, dans lequel la solution aqueuse est une solution d'hydroxyde de sodium ayant un pH d'environ 12,5.

7. Nanotubules d'un composé organique de formule : 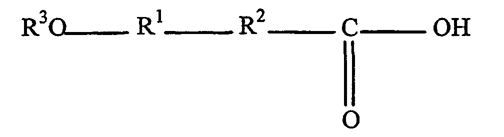 dans laquelle R¹ est un radical polycyclique à noyaux condensés en C₁₇ à C₂₀ comportant éventuellement un ou plusieurs substituants alkyle en C₁ à C₂₀, R² est un groupe alkylène linéaire ou ramifié en C₃ à C₂₀, R³ représente un atome d'hydrogène, un groupe alkyle en C₁ à C₂₀ ou un groupe aromatique en C₆ à C₃₀.

8. Nanotubules selon la revendication 7, dans lesquels R¹ est un groupe polycycliques de formule : 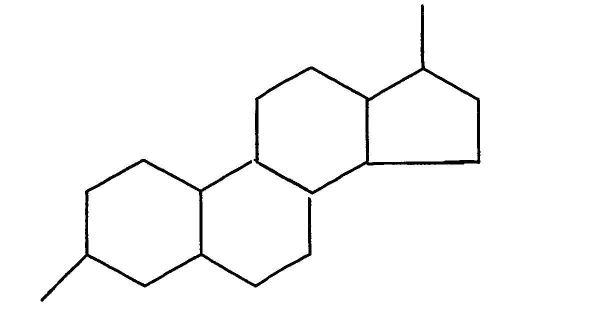 qui peut comporter un ou plusieurs substituants alkyle en C₁ à C₂₀.

9. Nanotubules d'acide lithocholique en milieu aqueux.

10. Utilisation des nanotubules organiques obtenus par le procédé selon l'une quelconque des revendications 1 à 6 pour la fabrication de nanotubules inorganiques par minéralisation.

11. Utilisation des nanotubules organiques obtenus par le procédé selon l'une quelconque des revendications 1 à 6 pour la fabrication de cathodes à émission de champ par métallisation des nanotubules.

12. Utilisation des nanotubules organiques obtenus par le procédé selon l'une quelconque des revendications 1 à 6 pour la fabrication de biocapteurs.

## Patentansprüche

1. Verfahren zur Herstellung von organischen Nanoröhrchen in einem wässrigen Medium, das die folgenden Stufen umfasst:
a) Herstellung einer basischen wässrigen Lösung, die einen pH-Wert von 12 bis 14, vorzugsweise von 12 bis 13,5, hat,
b) Zugabe einer organischen Verbindung mit der nachstehend angegebenen Formel zu der Lösung: 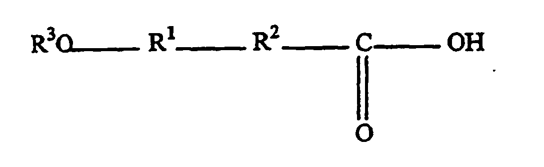 worin bedeuten:
R¹ einen polycyclischen C₁₇-C₂₀-Rest mit kondensierten Kernen, der gegebenenfalls einen oder mehrere C₁-C₂₀-Alkyl-Substituenten enthält,
R² eine lineare oder verzweigte C₃-C₂₀-Alkylengruppe und
R³ ein Wasserstoffatom, eine C₁-C₂₀- Alkylgruppe oder eine aromatische C₆-C₃₀-Gruppe, und
c) Rühren der Lösung für eine Zeitdauer, die ausreicht um stabile Röhrchen aus der organischen Verbindung in der Lösung zu bilden.

2. Verfahren nach Anspruch 1, worin R² steht für die Gruppe:

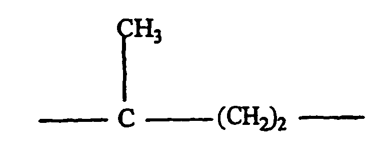

3. Verfahren nach einem der Ansprüche 1 und 2, worin R¹ steht für eine polycyclische Gruppe der Formel: 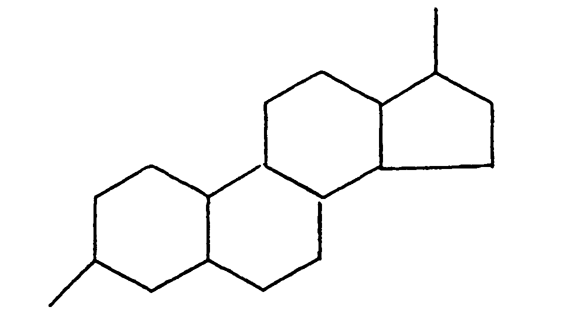 die ein oder mehrere C₁-C₂₀-Alkylsubstituenten tragen kann.

4. Verfahren nach Anspruch 1, worin die organischen Verbindung die Lithocholsäure der Formel ist:

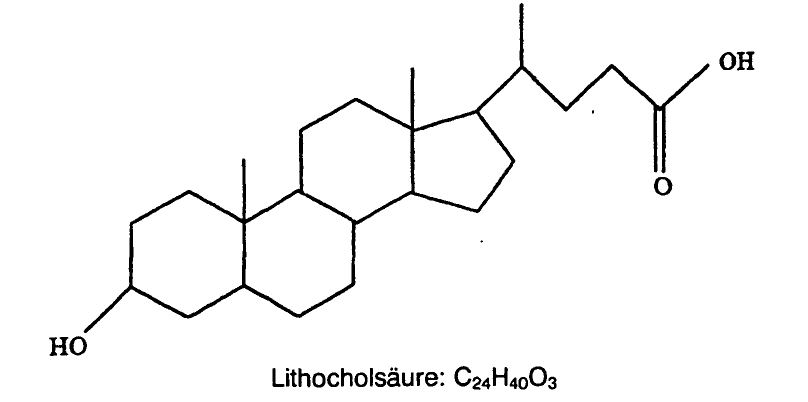

5. Verfahren nach einem der Ansprüche 1 bis 4, worin die wässrige Lösung eine Natriumhydroxid-Lösung ist.

6. Verfahren nach Anspruch 4, worin die wässrige Lösung eine Natriumhydroxid-Lösung mit einem pH-Wert von etwa 12,5 ist.

7. Nanoröhrchen aus einer organischen Verbindung der Formel: 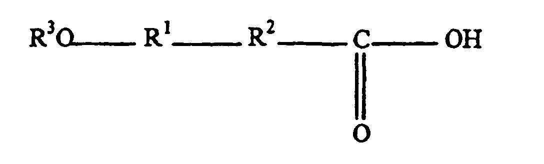 worin bedeuten:
R¹ einen polycyclischen C₁₇-C₂₀-Rest mit kondensierten Kernen, der gegebenenfalls einen oder mehrere C₁-C₂₀-Alkyl-Substituenten enthält,
R² eine lineare oder verzweigte C₃-C₂₀-Alkylengruppe,
R³ ein Wasserstoffatom, eine C₁-C₂₀- Alkylgruppe oder eine aromatische C₆-C₃₀-Gruppe.

8. Nanoröhrchen Anspruch 7, worin R¹ steht für eine polycyclische Gruppe der Formel: 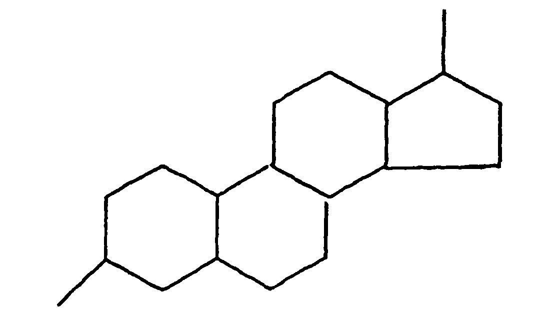 die einen oder mehrere C₁-C₂₀-Alkylsubstituenten tragen kann.

9. Nanoröhrchen aus Lithocholsäure in wässrigem Medium.

10. Verwendung der in dem Verfahren nach einem der Ansprüche 1 bis 6 erhaltenen organischen Nanoröhrchen zur Herstellung von anorganischen Nanoröhrchen durch Mineralisation.

11. Verwendung der in dem Verfahren nach einem der Ansprüche 1 bis 6 erhaltenen organischen Nanoröhrchen zur Herstellung von Feldemmisionskathoden durch Metallisierung der Nanoröhrchen.

12. Verwendung der in dem Verfahren nach einem der Ansprüche 1 bis 6 erhaltenen organischen Nanoröhrchen zur Herstellung von Biosensoren.

## Claims

1. A method for preparing organic nanotubules in an aqueous medium, comprising the following steps:
a) preparing a basic aqueous solution with a pH from 12 to 14, preferably from 12 to 13.5,
b) adding to the solution, an organic compound of formula: 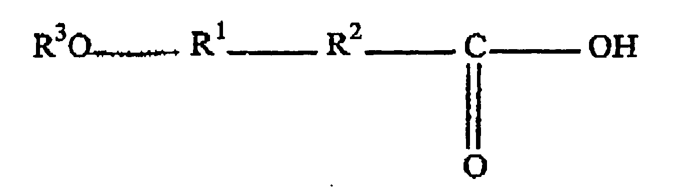 wherein R¹ is a C₁₇-C₂₀ polycyclic radical with fused rings, optionally including one or more C₁₇-C₂₀ alkyl substituents, R² is a C₃-C₂₀ linear or branched alkylene group, and R³ represents a hydrogen atom, a C₁-C₂₀ alkyl or C₆-C₃₀ aromatic group, and
c) submitting the solution to stirring for sufficient time in order to form stable tubules of the organic compound in the solution.

2. The method according to claim 1 wherein R² is the group:

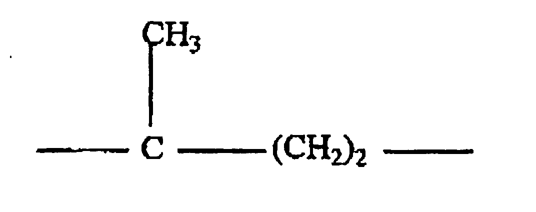

3. The method according to any of claims 1 and 2, wherein R¹ is a polycyclic group of formula: 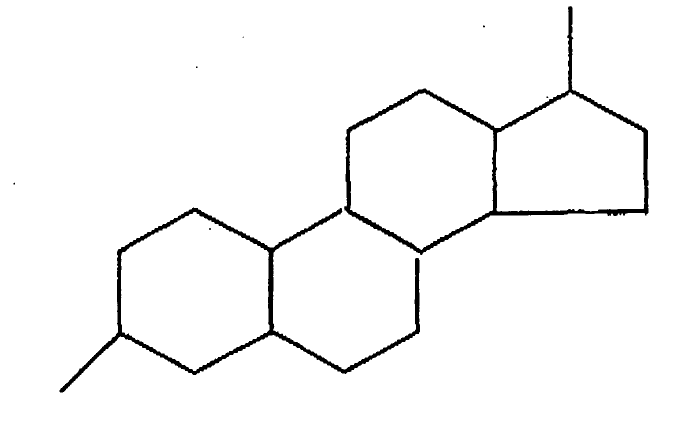 which may include one or more C₁ to C₂₀ alkyl substituents.

4. The method according to claim 1 wherein the organic compound is lithocholic acid of formula:

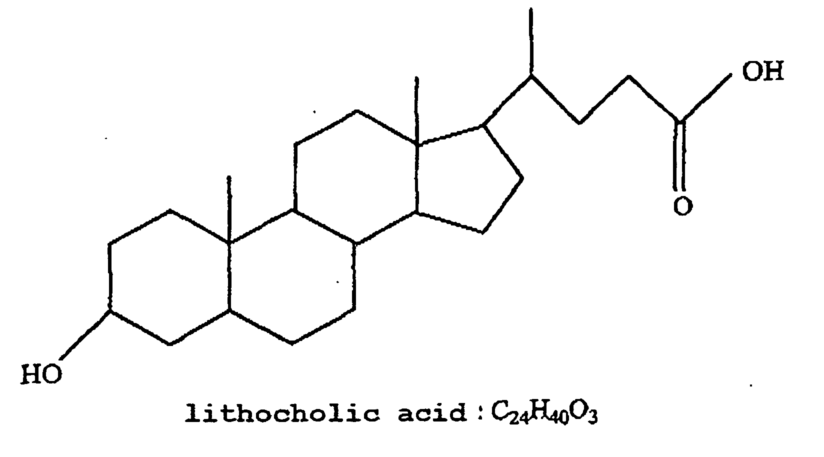

5. The method according to any of claims 1 to 4, wherein the aqueous solution is a sodium hydroxide solution.

6. The method according to claim 4, wherein the aqueous solution is a sodium hydroxide solution with a pH of about 12.5.

7. Nanotubules of an organic compound of formula: 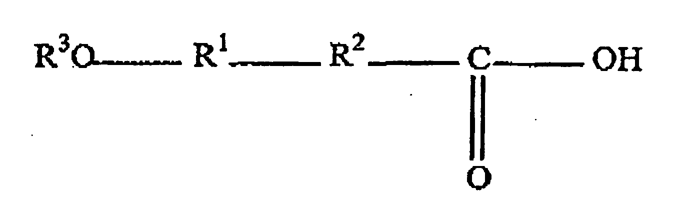 wherein R¹ is a C₁₇-C₂₀ polycyclic radical with fused rings, optionally including one or more C₁-C₂₀ alkyl substituents, R² is a C₃-C₂₀ linear or branched alkylene, R³ represents a hydrogen, a C₁-C₂₀ alkyl or a C₆-C₃₀ aromatic group.

8. The nanotubules according to claim 7, wherein R¹ is a polycyclic group of formula: 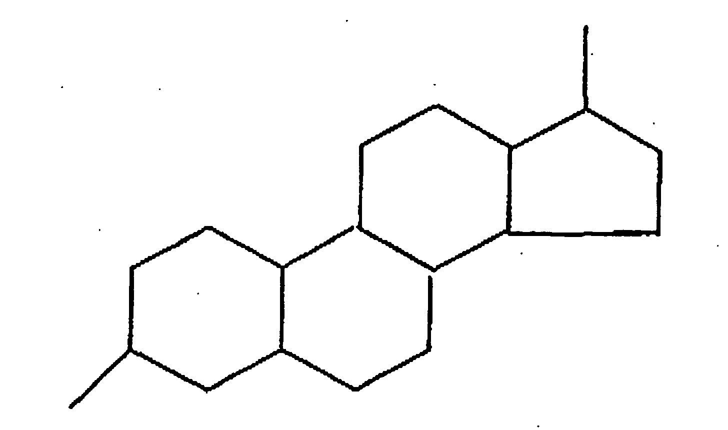 which may include one or more C₁-C₂₀ alkyl substituents.

9. Nanotubules of lithocholic acid in an aqueous medium.

10. Use of organic nanotubules obtained by the method according to any of claims 1 to 6 for manufacturing inorganic nanotubules by mineralization.

11. Use of organic nanotubules obtained by the method according to any of claims 1 to 6 for manufacturing field emission cathodes by metallization of nanotubules.

12. Use of organic nanotubules obtained by the method according to any of claims 1 to 6 for manufacturing biosensors.
